**Europäisches Patentamt**

**European Patent Office**

**Office européen des brevets**

(11) Veröffentlichungsnummer : **0 329 019 B1**

(12) **EUROPÄISCHE PATENTSCHRIFT**

(45) Veröffentlichungstag der Patentschrift :
**11.03.92 Patentblatt 92/11**

(51) Int. Cl.$^5$ : **A61F 2/30,** A61F 2/32, A61F 2/34

(21) Anmeldenummer : **89102254.3**

(22) Anmeldetag : **09.02.89**

(54) **Gelenkpfannenteil für eine Gelenkprothese.**

(30) Priorität : **11.02.88 DE 3804239**

(43) Veröffentlichungstag der Anmeldung :
**23.08.89 Patentblatt 89/34**

(45) Bekanntmachung des Hinweises auf die
Patenterteilung :
**11.03.92 Patentblatt 92/11**

(84) Benannte Vertragsstaaten :
**AT CH DE ES FR GB IT LI NL SE**

(56) Entgegenhaltungen :
**EP-A- 0 065 481**
**EP-A- 0 243 585**
**EP-A- 0 257 222**
**DE-A- 3 322 978**
**US-A- 4 298 993**

(73) Patentinhaber : **Howmedica GmbH**
**Professor-Küntscher-Str. 1-5**
**W-2314 Schönkirchen ü. Kiel (DE)**

(72) Erfinder : **Täger, Karl Heinrich, Prof. Dr.**
**Fichtenstrasse 1**
**W-8035 Gauting (DE)**

(74) Vertreter : **Dipl.-Ing. H. Hauck, Dipl.-Ing. E.**
**Graalfs, Dipl.-Ing. W. Wehnert, Dr.-Ing. W.**
**Döring**
**Neuer Wall 41**
**W-2000 Hamburg 36 (DE)**

EP 0 329 019 B1

**Beschreibung**

Die Erfindung betrifft ein Gelenkpfannenteil für eine Gelenkprothese, insbesondere ein Hüftgelenk-Pfannenteil einer Hüftgelenk-Endoprothese mit einem im Knochen befestigten Schalenelement, welches auf seiner Gelenkseite eine pfannenartig z.B. halbkugelförmig ausgebildete Vertiefung zur Aufnahme einer Gleitpfanne bzw. eines Inlays für einen Gelenkkopf aufweist.

Die feste Einfügung von einem Gelenkpfannenteil erfolgt vorwiegend durch Eindrehen des Pfannenkörpers in die Knochensubstanz mittels eines selbstschneidenden Gewindes, von dem beispielsweise mehrere Flügelabschnitte radial seitlich vom Pfannenkörper abstehen.

Es ist bekannt, die Oberschenkelteile von Hüftgelenk-Endoprothesen hohl und gelocht auszubilden (DE-OS 28 51 598). Der Hohlschaft bzw. seine Materialstärke sowie die Art und Verteilung der Löcher ist dabei der Längs- bzw. Biegefestigkeit des umgebenden Knochenbereiches anzupassen (EP-A 0 065 481). Die Löcher in dem Hohlkörper der Prothese ermöglichen das Einwachsen von Knochenmaterial, wodurch der Prothesenschaft einen zusätzlichen Halt bekommt. Der Hohlkörper kann ferner mit Knochengranulat zur Beschleunigung des Einwachsens der Prothese aufgefüllt werden. Der Schaft kann gemäß der EP-A-0 243 585 aus einem Guß-Formstück bestehen, wobei die Löcher in der frontalen und dorsalen Schaftseite angeordnet sind.

Der Erfindung liegt die Aufgabe zugrunde, ein Gelenkpfannenteil für eine Gelenkprothese zu schaffen, mit dem eine Verstärkung der Prothesenbefestigung am Knochen, eine einfache Anbringung der Prothese während der Operation und eine schnelle und sichere Einheilung erzielt wird.

Diese Aufgabe wird gelöst durch die Merkmale des des Patentanspruchs 1.

Damit ist dem Gelenkpfannenteil einer Gelenkprothese erfindungsgemäß eine Struktur gegeben, in die einerseits während der Operation lose Knochensubstanz eingefüttert werden kann, ohne sich bei der anschließenden Handhabung herauszubewegen und durch welche andererseits das Innere des Gelenkpfannenteils über eine mehrfach verzweigte bzw. vernetzte Brückenbildung verstärkt mit den angrenzenden Knochenpartien verankert wird.

Nach einer Ausgestaltung der Erfindung sind radial bzw. quer zwischen den beiden Schalenteilen verlaufende Abstützungen gemäß Patentanspruch 4 vorgesehen. Diese Abstützungen tragen also die Elemente des dem Knochen zugewandten Schalenteils.

Obwohl die Öffnungen jede beliebige Querschnittsform besitzen können, dürften runde oder längliche Löcher fertigungstechnisch am günstigsten sein.

Dabei kann die Anordnung der Öffnungen gemäß den Patentansprüchen 5 bis 8 von speziellem Vorteil sein, wobei auch eine besondere z.B. festigkeitsoptimierte Orientierung der Öffnungen - auch nach ihrer Form - gegeben sein kann.

Durch die erfindungsgemäß erzielte verstärkte Verwachsung der Prothese mit dem Knochen kann auch die Größe und Anzahl der Flächenteile bzw. Flügel des selbstschneidenden Gewindes vermindert und die Operationsarbeit verbessert werden.

Das erfindungsgemäße Schalenelement wird vorzugsweise einteilig geformt, beispielsweise im Gießverfahren. Die Schalen können jedoch einteilig getrennt geformt und anschließend miteinander verbunden werden, beispielsweise durch Schweißung.

Um eine möglichst große Verbindungsfläche zwischen der in den Zwischenraum des Schalenelements eingebrachten Knochensubstanz und dem Hüftknochen zu erhalten, ist es zweckmäßig, relativ große bzw. langgestreckte Öffnungen vorzunehmen. Daher werden nach Möglichkeit mehrere Reihen langgestreckter Öffnungen in Umfangsrichtung angeordnet, wobei die Lochreihen in einer Richtung senkrecht dazu einen Abstand voneinander aufweisen, in dem zum Beispiel die Abschnitte eines selbstschneidenden Gewindes geformt sind. Eine Möglichkeit besteht darin, die einzelnen langgestreckten Öffnungen oder Löcher auf Lücke anzuordnen. Vorzuziehen ist indessen eine Anordnung, bei der die langgestreckten Öffnungen jeweils auf einer Linie enden, die einer Fallinie an der Außenseite des Schalenelements entspricht. Zweckmäßigerweise enden dann auch die Gewindeabschnitte in Höhe der Lochkanten. Dadurch ergibt sich zwischen den Gewindeabschnitten eine gerade Nut, so daß die auf einer Linie endenden Gewindeabschnitte eine wirksame Gewindeschneidfunktion entfalten können, vergleichbar einem herkömmlichen Gewindeschneider.

Das erfindungsgemäße Schalenelement wird mit Hilfe der selbstschneidenden Gewindeabschnitte in eine vorgeformte Höhlung im Hüftknochen eingeschraubt. Hierfür wird zweckmäßigerweise ein geeignetes Drehwerkzeug verwendet. Zum Angriff für ein Drehwerkzeug sieht eine Ausgestaltung der Erfindung vor, daß das Schalenelement auf der dem Hüftknochen abgewandten Seite mindestens zwei diametral angeordnete Ausnehmungen aufweist für die Aufnahme eines Drehwerkzeugs.

Das Inlay, das vorzugsweise aus Kunststoff besteht, soll auf einfache Weise in der Innenschale anbringbar sein. Hierzu sieht eine Ausgestaltung der Erfindung vor, daß die Innenschale an der dem Hüftknochen zugewandten Seite eine Aussparung oder einen zur Außenschale geöffneten Durchgang aufweist mit einem Hin-

2

terschnitt, wobei das Inlay auf der Außenseite einen Rastabschnitt aufweist, der mit der Aussparung bzw. dem Hinterschnitt zusammenwirkt. Vorzugsweise besteht eine koaxiale, zur Außenseite des Schalenelements hindurchgehende Öffnung mit einem Hinterschnitt, wobei das Inlay einen koaxialen zylindrischen Zapfen aufweist mit einer Reihe von Nasen, die den Hinterschnitt im Durchgang hintergreifen. Auf diese Weise kann das Inlay einfach in die Innenschale eingeschnappt und auch relativ leicht wieder gelöst werden, insbesondere durch einen Druck von der Außenseite des Schalenelements.

Gemäß den Patentansprüchen 11 und 12 kann das Schalenelement eine unterschiedliche Außenform aufweisen, wobei die Anordnung der beiden Schalenteile speziell nach Patentanspruch 13 erfolgen kann.

Als Material für das Schalenelement kommt erprobt gewebefreundliches Material, insbesondere hochlegiertes Metall, wie Titan, Vitallium etc. in Frage. Für die im Schalenelement gelagerte Gleitschale bzw. als Inlay kommt ein High-Density Polyethylen mit einer Wandstärke von etwa 5 mm zur Anwendung, obwohl in vielen Fällen auch diese Gleitschale aus Metall bestehen kann.

Als eingefütterte Knochensubstanz hat sich sowohl körpereigenes als auch körperfremder (bankkonservierter) Knochenspan bzw. Knochengewebe bewährt.

Mit der Erfindung werden derartige Endoprothesen in zum Körper hin weitgehend offener Form ausgeführt, so daß neben einem schnellen Einwachsen auch eine spätere körpereigene Versorgung der gewachsenen Knochenbereiche verbessert gewährleistet ist.

In besonders bevorzugter Form kann die Gelenkpfanne auch dadurch vereinfacht ausgebildet sein, daß das Schalenelement das dem Knochen zugewandte, mit Öffnungen versehene Schalenteil aufweist und als pfannenseitiges Schalenteil die innenliegende Oberfläche der Gelenk- und Gleitpfanne vorgesehen ist, wobei der freie Zwischenraum zwischen diesen beiden Teilen vorgesehen ist. Gemäß Patentanspruch 14 können weitere material- und raumsparende Ausführungsformen vorteilhaft sein.

Auch dabei liegt die radiale Dicke des Zwischenraums bzw. die Dicke des Netzgittergewebes zwischen etwa 5 - 10 mm.

Insbesondere kann mit diesen Ausführungsformen ein größerer Gleitpfannendurchmesser zum Einsatz kommen. Eine damit auch mögliche Vergrößerung des Gelenkkugeldurchmessers verringert die auf den Oberschenkelteil ausgeübten Kräfte, so daß die Kombination gemäß Patentanspruch 18 synergistische Vorteile bietet.

Danach ist das erfindungsgemäße Gelenkpfannenteil mit einem für eine Hüftgelenk-Endoprothese verwendbaren Oberschenkelteil kombiniert, dessen Schaft einen Innenraum und damit in Verbindung stehende Öffnungen seiner Wandung aufweist. Der Schaft kann als Guß-Formstück ausgeführt sein, so daß sich einerseits keine Festigkeitsprobleme ergeben und andererseits der Halt der losen eingefütterten Knochensubstanz verbessert ist sowie sich zudem keine Halterungsprobleme auch durch ein dem Markkanal allseitig voll angepaßtes Außenprofil ergeben.

Die Erfindung ist nachstehend in Ausführungsbeispielen näher erläutert. Es zeigen:

Fig. 1 und 2 schematische Schnittdarstellungen einer Hüftgelenk-Endoprothese von der Frontseite her gesehen,

Fig. 3 eine von der Knochensubstanz her gesehene Draufsicht auf einen Teil des erfindungsgemäßen Schalenelements,

Fig. 4 einen Querschnitt längs der Schnittlinie IV-IV in Fig. 1.

Fig. 5 eine schematische Querschnittsdarstellung durch ein erfindungsgemäßes Schalenelement und

Fig. 6 A und B auf zwei Seiten einer Mittelebene E dargestellt Querschnittsdarstellungen je einer weiteren Ausführungsform des erfindungsgemäßen Schalenelements

Fig. 7 eine Draufsicht auf den äußeren Schalenteil eines Schalenelements nach der Erfindung,

Fig. 8 einen Schnitt durch die Darstellung nach Fig. 7 entlang der Linie VIII-VIII,

Fig. 8A eine Ansicht in Richtung des Pfeils VIIIA, jedoch nur einen Teil der Außenseite des äußeren Schalenteils,

Fig. 9 eine Seitenansicht einer abgewandelten Ausführungsform eines Schalenteils nach Fig. 7,

Fig. 9a eine Draufsicht auf ein Schalenelement mit durchgehendem Gewindegang,

Fig. 10 einen Schnitt durch ein Inlay zur Aufnahme in einer Innenschale des Schalenelements nach Fig. 8,

Fig. 11 eine Draufsicht auf das Inlay nach Fig. 10,

Fig. 12 und Fig. 13 eine Endansicht bzw. eine Seitenansicht, teilweise im Schnitt, eines Werkzeugs zum Einschrauben eines Schalenelements nach Fig. 8, 8A.

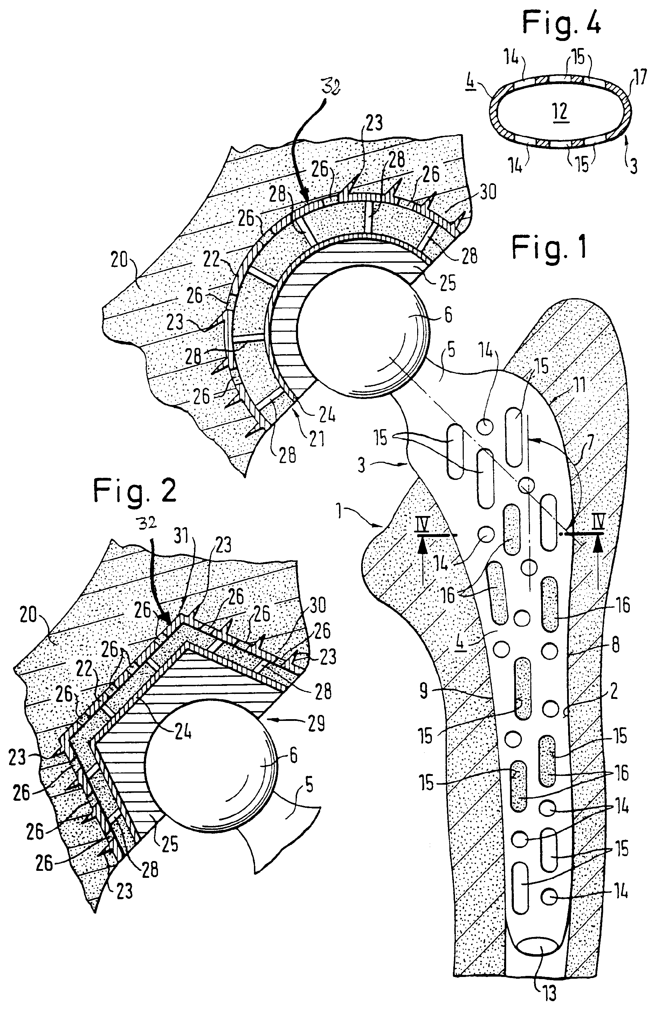

Am hüftseitigen Ende 1 eines Oberschenkelknochens ist in dessen Markkanal 2 der Schaft 4 des Oberschenkelteils 3 eines künstlichen Hüftgelenks eingebracht (Fig. 1), welcher auf einem sich verjüngenden Hals 5 eine Gelenkkugel 6 trägt, wobei zwischen der Schaft- und der Halslängsachse ein Winkel 7 von etwa 135° liegt.

Der Schaft 4 besitzt eine laterale 8 und eine mediale Seite 9 und weist auf seiner frontalen und dorsalen Seite Öffnungen auf, die in unterschiedlicher Form, Größe, Anzahl und Verteilung vorliegen und mit einem im Inneren des Schafts 4 vorgesehenen Innenraum 12 in Verbindung stehen. Die Art der Anbringung der Öffnungen, runde Öffnungen 14 und langgestreckte Öffnungen 15, gewährleistet nicht nur ein Hineinbringen von körpereigener oder körperfremder Knochensubstanz bzw. entsprechendem Gewebe 16 auch in den hohlen Innenraum 12, sondern auch deren Halt während der mit kräftigen Bewegungen und auch Stößen verbundenen Einsetzoperation der Prothese.

Auch zu diesem Zwecke ist der Schaft 4 als Guß-Formstück z.B. aus Tantal hergestellt; wodurch sich eine vergleichsweise steife Gußstückwandung 17 ergibt, die in den Formübergängen, z.B. auch um die Öffnung 13 im distalen Ende des Schafts zusätzliche Versteifungsdicken ermöglicht.

Im Hüftknochen 20 ist eine Endoprothesen-Pfanne 21 befestigt, die mit ihrem Schalenelement 32 gemäß den Fig. 1 und 2 unterschiedliche Form besitzen kann, gemäß Fig. 1 als Halbkugel-Schalen bzw. Halbkugelstumpf-Schalen und in Fig. 2 als Kegelstumpfform 29 vorkommen kann.

Das Schalenelement 32 besitzt gemäß Fig. 1, 2 und 5 ein knochenseitiges Schalenteil 22 (Innenschale) und ein pfannenseitiges Schalenteil 24 (Außenschale), an dem auch die Flügel eines selbstschneidenden Gewindes 23 angebracht sind, mit denen die Pfanne 21 in den weichen Beckenknochenbereich 30 unter Kraftanwendung eingedreht wird.

Im Zwischenraum 27 sind Stütz- und Distanzzapfen 28 bzw. Verbindungen vorgesehen.

Durch Öffnungen 26 bzw. entsprechende Perforationen wird vor dem Einsetzen ebenfalls Knochensubstanz in den Zwischenraum 27 eingefüttert, welche später mit dem Hüftknochen 20 zusammenwächst und die Gelenkpfanne 21 mittels Knocheneinwachsungen bzw. Knochenbrücken fest im Hüftknochen 20 verankert.

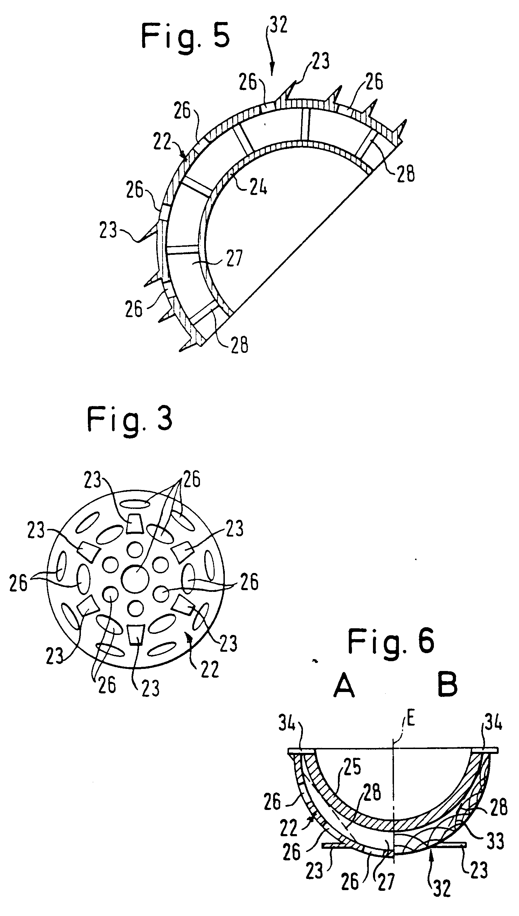

Die Öffnungen 26 sind (Fig. 3) über die Fläche des knochenseitigen Schalenteils 22 verteilt, von der auch die Flügel des Gewindes 23 nach außen abstehen.

In der konkaven Hohl-Fläche des pfannenseitigen Schalenteils 24 ist die eigentliche Gelenkgleitpfanne 25 eingebracht, und zwar unter Befestigung am Randbereich des Schalenteils 22 in der sich die Gelenkkugel 6 bewegt.

In den besonderen Ausführungsformen gemäß Fig. 6 A kann das pfannenseitige Schalenteil 24 des Schalenelements 32 auch von der Außenseite der Gelenkgleitpfanne 25 gebildet werden, wobei die Knochensubstanz 16 auf noch einfachere Weise in den Zwischenraum 27 eingefüttert werden kann und nach vollzogener Heilung körpereigene Knochensubstanz die Gelenkgleitpfanne 25 abstützt. Im Falle der Fig. 6 können die Stützt- und Distanzzapfen 28 als Verstrebungen zwischen dem gegebenenfalls versteiften Randbereich 34 des Schalenteils 22 und dessen Boden verlaufen. Dieser wirken nach der Einheilung auch Torsionskräfte entgegen.

Bei der speziellen Ausführungsform gemäß Fig. 6 B ist der knochenseitige Schalenteil 22 weitgehend in ein Netzgittergeflecht 33 aufgelöst, welches von der Ebene des Schalenteils 22 getragen von einer Netzgitter-Konstruktion bis in den Zwischenraum 27 reicht oder diesen auch ausfüllen kann.

Als Netzgitterelemente können feste Metalldrähte oderbänder bzw. -streifen vorgesehen sein, um die die Knochensubstanz herumwächst, wodurch eine flache Verankerung der Pfanne über die Dicke des Zwischenraums 27 erreicht wird.

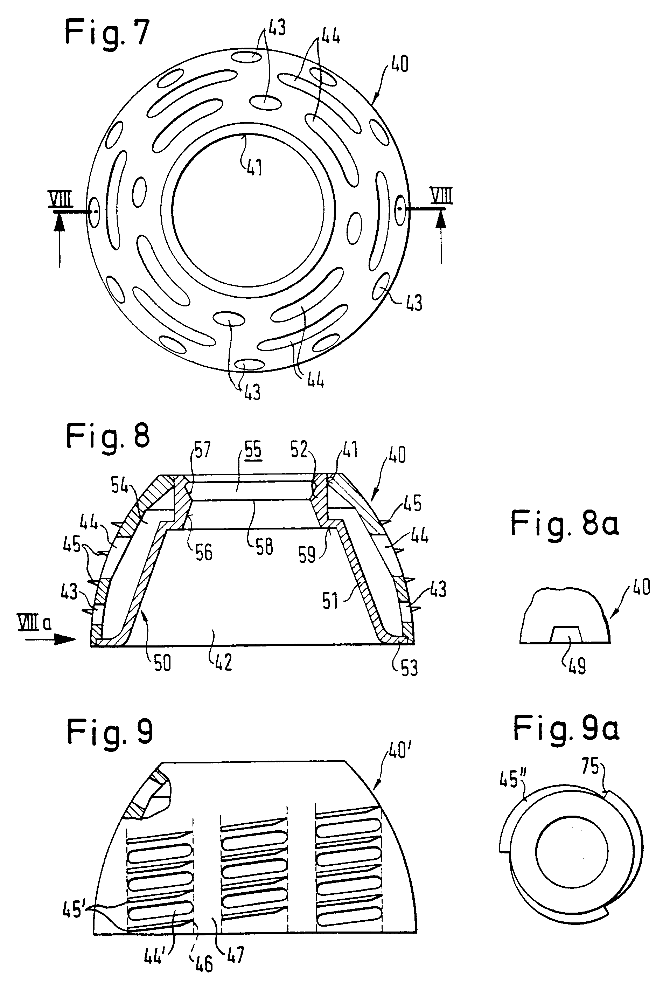

Bei der Ausführungsform eines Schalenelements nach den Figuren 7 bis 9 ist zu erkennen, daß ein äußeres Schalenteil 40 annähernd als Kugelsegment geformt ist (siehe insbesondere Fig. 8) mit einer kreisrunden Öffnung 41 an dem dem Hüftknochen zugewandten Ende, welche konzentrisch ist zur Eintrittsöffnung 42 des Schalenteils 40. Bei der Ausführungsform nach Fig. 7 weist das Schalenteil 40 mehrere Reihen von Löchern auf, wobei kreisförmige Löcher 43 mit langgestreckten Löchern 44 abwechseln (der Öffnung 41 benachbarte Lochreihe, während die mittlere Lochreihe nur aus langgestreckten Öffnungen 44 und die äußere Lochreihe in Fig. 7 aus kreisförmigen Löchern 43 besteht). Zwischen den Lochreihen sind Abschnitte eines Gewindes vorgesehen (nicht gezeigt) ähnlich den Flügeln 23 der oben beschriebenen Figuren. In Fig. 8 sind indessen die Gewindeabschnitte bei 45 gezeigt. Bei der Ausführungsform nach Fig. 9, welche ein Schalenteil 40 zeigt, welches in seiner Form derjenigen nach Fig. 7 und 8 gleicht, sind ausschließlich in Umfangsrichtung mehrere Reihen von langgestreckten Öffnungen 44′ vorgesehen, die entlang Linie 46 enden, die annähernd senkrecht zur Schraubenlinie verlaufen, entlang der die Öffnungen 44′ angeordnet sind. Zwischen den Öffnungen 44′ befinden sich Gewindeabschnitte 45′, die ebenfalls an der Linie 46 enden. Dadurch wird zwischen den Gewindeabschnitten 45′ eine freie Nut 47 erhalten, die wie eine Nut eines Gewindeschneiders wirkt. Die Ausführungsform nach Fig. 9 läßt sich daher besonders wirksam in den Hüftknochen einschrauben. Fig. 9a zeigt eine weitere Ausführungsform in der die Gewindeabschnitte als Abschnitte mit durchgehend verlängertem Gewindeabschnitt 75 ausgeführt sind, der an seinem zur Drehrichtung hinteren Ende stetig zum Schalenteil 40 zurücktritt.

Das innere Schalenteil 50 bei der Ausführungsform nach Fig. 8 besteht aus einem konischen Abschnitt 51 und einem sich daran anschließenden zylindrischen Abschnitt 52. Der konische Abschnitt 51 weist einen radialen Flansch 53 auf, der sich zum Schalenteil 40 hin erstreckt. Werden die Schalenteile 40, 50 getrennt geformt, wird der Flansch in einer Umfangsausnehmung des Schalenteils 40 versenkt angeordnet (wie in Fig. 8 gezeigt). Werden hingegen die beiden Schalenteile 50, 40 z.B. durch Gießen einteilig geformt, ist der Flansch 53 einteilig mit dem entsprechenden Abschnitt des Schalenteils 40 verbunden. Der zylindrische Abschnitt 52 greift in die Öffnung 41 ein und ist bei einer einteiligen Formung des Schalenelements in dem Öffnungsbereich einteilig mit dem äußeren Schalenteil 40 verbunden. Bei einer Formung des Schalenelements aus zwei getrennten Teilen greift der zylindrische Abschnitt 52 in die Öffnung 41 ein und wird in diesem Bereich, z.B. durch Schweißung, mit dem Schalenteil 40 fest verbunden.

Zwischen den Schalenteilen 40, 50 ergibt sich ein ringförmiger konischer Zwischenraum 54, in den über die Löcher 43, 44 Knochensubstanz eingefüllt werden kann.

Das Schalenteil 50 weist einen zur Öffnung 41 des äußeren Schalenteils 40 sich erstreckenden Durchgang 55 auf. Dieser weist einen zur Öffnung 42 gerichteten konischen Abschnitt 56 auf, an den sich ein entgegengesetzt konischer Abschnitt 57 anschließt. Der konische Abschnitt 57 endet in einem Radius, wodurch sich der Durchgang 55 nach außen hin wieder verengt.

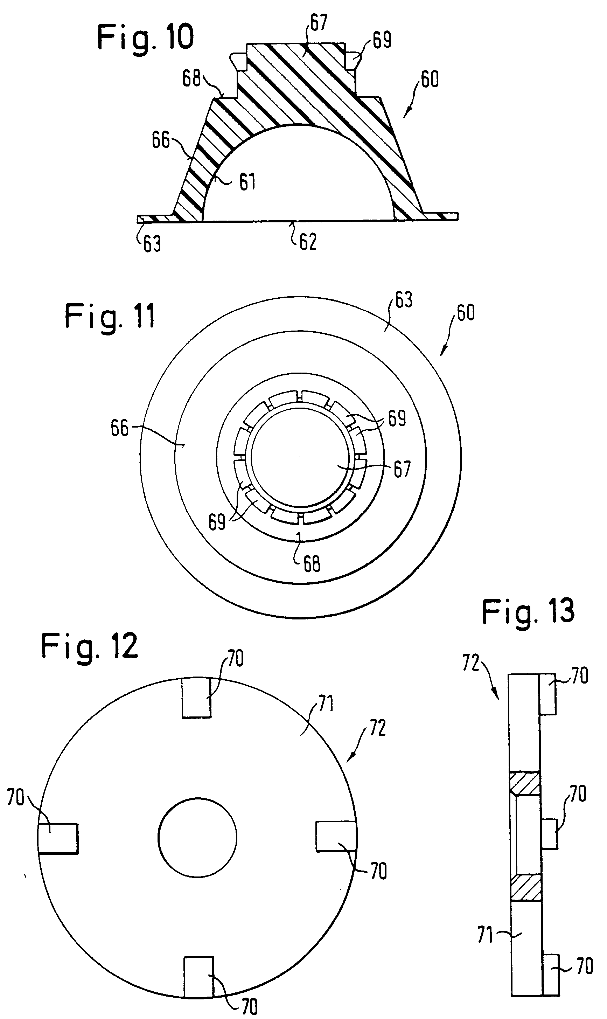

Fig. 10 und 11 zeigen eine Gelenkschale bzw. ein Inlay 60 aus einem geeigneten Kunststoffmaterial. Es besitzt eine halbkugelige Höhlung 61 für die Aufnahme einer Gelenkkugel (siehe auch obige Ausführungsform). In Höhe der Eintrittsöffnung 62 für die halbkugelige Höhlung 61 weist das Inlay 60 einen radialen Flansch 63 auf, der sich beim Einsetzen des Inlays 60 in das äußere Schalenteil 50 gegen die Unterseite des Flansches 53 anlegt. Außen ist das Inlay 60 bei 66 konisch ausgebildet. Der konische Abschnitt 66 kann passend in den konischen Abschnitt 51 des inneren Schalenteils 50 eingesetzt werden. Das Inlay 60 besitzt außerdem auf der der Eintrittsöffnung 62 gegenüberliegenden Seite einen im wesentlichen konischen Zapfen 67, der mit dem konischen Abschnitt 66 eine Schulter 68 bildet. Oberhalb der Schulter sind mehrere sich schräg nach außen erstreckende in Umfangsrichtung auf Abstand zueinander angeordnete Nasen 69 vorgesehen (siehe auch Fig. 11). Beim Einsetzen des Inlays 60 in das innere Schalenteil 50 stößt die Schulter 68 gegen eine Schulter 59 des Schalenteils 50 im Übergangsbereich zwischen dem konischen Abschnitt 51 und dem zylindrischen Abschnitt 52. Außerdem gleiten die Nasen 69, sich vorübergehend verformend, über den Engquerschnitt 58 zwischen den konischen Abschnitten 56, 57 des Durchgangs 55, um mit dem konischen Abschnitt 57 anschließend zusammenzuwirken. Dadurch ist das Inlay 60 sicher im Schalenteil 50 verriegelt und kann nur durch einen entsprechenden Druck über die Öffnung 41 aus dem Schalenteil 50 entfernt werden. Die Stirnseite des Inlays 60 bzw. des Zapfens 67 liegt annähernd in Höhe der Außenseite des äußeren Schalenteils 40 im Bereich der Öffnung 41.

Wie aus Fig. 8a hervorgeht, sind in der Stirnseite des äußeren Schalenteils 40 auf der der Gelenkkugel zugewandten Seite Aussparungen 49 geformt. Insgesamt sind vier im Umfangsabstand von 90° angeordnete Aussparungen 49 vorgesehen. Sie dienen gemäß Fig. 12 zur Aufnahme von Vorsprüngen 70, die an einem scheibenförmigen Teil 71 eines Werkzeugs 72 geformt sind. Die Scheibe 71 kann mit einem geeigneten Drehwerkzeug verbunden werden, das von Hand oder motorisch betätigt wird. Mit Hilfe des Werkzeugs 72 kann das Schalenelement nach Fig. 8, ggf. zusammen mit dem Inlay nach Fig. 10 in die vorbearbeitete Höhlung der Hüftknochen eingedreht werden.

## Patentansprüche

1. Gelenkpfannenteil (21) für eine Gelenkprothese, insbesondere Hüftgelenk-Pfannenteil einer Hüftgelenk-Endoprothese mit einem im Hüftknochen (20) befestigten Schalenelement (32; 40 und 50), welches auf seiner Gelenkseite eine pfannenartig z.B. halbkugelförmig ausgebildete Vertiefung zur Aufnahme einer Gleitpfanne (25) bzw. eines Inlays (60) für das Gelenk aufweist, dadurch gekennzeichnet, daß das Schalenelement (32; 40 und 50) aus mindestens zwei radial auf Abstand mit einem freien Zwischenraum (27; 54) zueinander angeordneten Schalenteilen (22, 24; 40, 50) aufgebaut ist, von denen mindestens in dem dem Hüftknochen (20) zugewandten Schalenteil (Innenschale 22, 40) Öffnungen (26; 43, 44) vorgesehen sind, deren Anzahl, Verteilung, Form und Größe während der Operation die Einlagerung und sichere Halterung von Spongiosa bzw. loser Knochensubstanz (16) in den Zwischenraum (27; 54) ermöglicht und danach ein Hineinwachsen von neuer, fester Knochensubstanz gewährleistet.

2. Gelenkpfannenteil nach Anspruch 1, dadurch gekennzeichnet, daß das Schalenelement (32; 40 und 50) einteilig geformt ist.

3. Gelenkpfannenteil nach Anspruch 1, dadurch gekennzeichnet, daß die Schalenteile (22, 24; 40, 50) einteilig geformt sind und passend ineinandergesteckt und anschließend miteinander verbunden werden, vorzugsweise durch Schweißung.

4. Gelenkpfannenteil nach einem der Ansprüche 1 bis 3, dadurch gekennzeichnet, daß zwischen den bei-

EP 0 329 019 B1

den Schalenteilen (22, 24) vorzugsweise radiale Stütz- bzw. Distanzzapfen (28) vorgesehen sind.

5. Gelenkpfannenteil nach einem der Ansprüche 1 bis 4, dadurch gekennzeichnet, daß die Öffnungen (26; 43) in dem dem Hüftknochen (20) zugewandten Schalenteil (22; 40) kreisrund sind.

6. Gelenkpfannenteil nach einem der Ansprüche 1 bis 4, dadurch gekennzeichnet, daß die Öffnungen (26; 44) in dem dem Knochen (20) zugewandten Schalenteil (22; 40) in Umfangsrichtung langgestreckt sind.

7. Gelenkpfannenteil nach Anspruch 6, dadurch gekennzeichnet, daß mehrere in Umfangsrichtung verlaufende Reihen von langgestreckten Öffnungen (44) vorgesehen sind und die Öffnungen (44) einer Reihe gegenüber denen der anderen Reihe versetzt angeordnet sind.

8. Gelenkpfannenteil nach Anspruch 6, dadurch gekennzeichnet, daß mehrere in Umfangsrichtung verlaufende Reihen von langgestreckten Öffnungen (44') vorgesehen sind und die Öffnungen (44') aller Reihen in Achsrichtung ausgerichtet sind.

9. Gelenkpfannenteil nach einem der Ansprüche 1 bis 8, dadurch gekennzeichnet, daß zwischen den Öffnungen (43, 44, 44') Abschnitte (45, 45') eines selbstschneidenden Gewindes an der Außenseite des Schalenelements geformt ist.

10. Gelenkpfannenteil nach Anspruch 8 und 9, dadurch gekennzeichnet, daß das Gewinde durch Aussparungen unterbrochen ist derart, daß die Gewindeabschnitte in den Aussparungen auf einer Linie (46) enden.

11. Gelenkpfannenteil nach einem der Ansprüche 1 bis 10, dadurch gekennzeichnet, daß die Schalenteile (22, 24) kugelabschnittförmig sind.

12. Gelenkpfannenteil nach einem der Ansprüche 1 bis 10, dadurch gekennzeichnet, daß die Schalenteile (22, 24) kegelstumpfförmig sind.

13. Gelenkpfannenteil nach einem der Ansprüche 1 bis 12, dadurch gekennzeichnet, daß die Schalenteile (22, 24; 40, 50) konzentrisch angeordnet sind.

14. Gelenkpfannenteil nach einem der Ansprüche 1 bis 13, dadurch gekennzeichnet, daß das dem Knochen (20) zugewandte Schalenteil (22) als Netzgittergeflecht (33) ausgebildet ist, welches den Zwischenraum (27) mindestens teilweise ausfüllt sowie vom Knochen durchwachsbar ist und dessen einzelne Gitter- bzw. Netzgeflechtelemente fest gegeneinander abgestützt sind, vorzugsweise an den Kreuzungspunkten fest miteinander verbunden sind.

15. Gelenkpfannenteil nach einem der Ansprüche 1 bis 14, dadurch gekennzeichnet, daß das Schalenelement (40) auf der dem Hüftknochen (20) abgewandten Stirnseite mindestens zwei diametral angeordnete Ausnehmungen (49) aufweist für die Aufnahme eines Drehwerkzeugs (72).

16. Gelenkpfannenteil nach einem der Ansprüche 1 bis 15, dadurch gekennzeichnet, daß die Innenschale (50) an der dem Hüftknochen zugewandten Seite eine Aussparung oder einen zur Außenschale (40) geöffneten Durchgang (55) aufweist mit einem Hinterschnitt (57) und das Inlay (60) auf der Außenseite einen Rastabschnitt (67, 69) aufweist, der mit der Aussparung bzw. dem Hinterschnitt (57) zusammenwirkt.

17. Gelenkpfannenteil nach Anspruch 16, dadurch gekennzeichnet, daß der aus Kunststoff bestehende Inlay (60) einen annähernd zylindrischen Zapfen (67) aufweist, der an der Außenseite mehrere in Umfangsrichtung beabstandete Nasen (69) aufweist, die mit dem Hinterschnitt (57) in dem Durchgang (55) zusammenwirken.

18. Gelenkpfannenteil nach einem der vorhergehenden Ansprüche 1 bis 17, dadurch gekennzeichnet, daß das Gelenkpfannenteil (21) kombiniert ist mit einem Oberschenkelteil (3) einer Gelenk- bzw. Hüftgelenk-Endoprothese, wobei im Schaft (4) des Oberschenkelteils (3) ein Innenraum (12) sowie Öffnungen (14, 15) zwischen der Außenseite des Schafts und dessen Innenraum vorgesehen ist, wobei die Öffnungen (14, 15) allein in der frontalen Seite (10) und/oder dorsalen Seite (11) des Schafts (4) angeordnet sind, wobei die Öffnungen (14, 15) und der Innenraum (12) in Form und Größe während der Operation die Einlagerung und Halterung von Spongiosa (16) bzw. loser Knochensubstanz in den bzw. im hohlen Innenraum (12) und danach ein Hineinwachsen von neuer, fester Knochensubstanz gewährleisten, wobei der Schaft (4) aus einem Guß-Formstück besteht, dessen massive vergleichsweise dicke Wandung (17) für die eingefütterte Knochensubstanz auch innerhalb der Öffnungen (14, 15) einen Stützhalt gewährleistet, und wobei die Außenseite des Schafts (4) mit seinem Profil dicht an das zuzuordnende Innen-Profil des Markkanals (2) angepaßt ausgebildet ist.

## Claims

1. An acetabular cup (21) for a joint prosthesis, particularly hip joint socket member of a hip joint endoprosthesis, having a cup element (32; 40 and 50) which is affixed to the hip bone (20) and has a cup-shaped, e.g. hemispherical recess which is arranged on its joint side for accommodating a sliding socket (25) or an inlay (60) for the joint, characterized in that the cup element (32; 40 and 50) is made up from at least two cup portions (22, 24; 40, 50) being spaced radially from each other to define a free intermediate space (27; 54) therebetween,

6

wherein at least the cup portion (inner cup 22, 40) facing the hip bone (20) has openings (26; 43, 44), the number, distribution, shape and size thereof permitting spongiosa or loose bone material (16) to be introduced and securely held in the intermedidate space (27; 54) during operation and thereafter, ensuring growth thereinto of new, strong bone material.

2. The acetabular cup according to claim 1, characterized in that the cup element (32; 40 and 50) is integrally formed.

3. The acetabular cup according to claim 1, characterized in that the cup portions (22, 24; 40, 50) are integrally formed and fitted in one another and thereafter intercoupled, preferably through welding.

4. The acetabular cup according to one of the claims 1 to 3, characterized in that preferably radial support and spacer pins (28) are provided between both cup portions (22, 24).

5. The acetabular cup according to one of the claims 1 to 4, characterized in that the openings (26; 43) in the cup portion (22; 40) facing the hip bone (20) are round.

6. The acetabular cup according to one of the claims 1 to 4, characterized in that the openings (26; 44) in the cup portion (22; 40) facing the bone (20) are elongated in circumferential direction.

7. The acetabular cup according to claim 6, characterized in that a plurality of rows of elongated openings (44) arranged in circumferential direction are provided, and the openings (44) of one row are displaced to the openings of the other row.

8. The acetabular cup according to claim 6, characterized in that a plurality of rows of elongated openings (44') arranged in circumferential direction are provided, and the openings (44') of all rows are oriented in axial direction.

9. The acetabular cup according to one of the claims 1 to 8, characterized in that portions (45, 45') of a self-tapping thread are provided on the outside of the cup element between the openings (43, 44, 44').

10. The acetabular cup according to claims 8 and 9, characterized in that the thread is interrupted by recesses such that the thread portions in the recesses terminate along a line (46).

11. The acetabular cup according to one of the claims 1 to 10, characterized in that the cup portions (22, 24) are formed like spherical segments.

12. The acetabular cup according to one of the claims 1 to 10, characterized in that the cup portions (22, 24) are of frusto-conical shape.

13. The acetabular cup according to one of the claims 1 to 12, characterized in that the cup portions (22, 24; 40, 50) are concentrically arranged.

14. The acetabular cup according to one of the claims 1 to 13, characterized in that the cup portion (22) facing the bone (20) is formed like mesh network arrangement (33) which at least partially fills the intermediate space (27) and through which the bone tissue can grow, and the individual mesh or network elements of the arrangement are firmly supported against each other, preferably being firmly interconnected at the points of intersection.

15. The acetabular cup according to one of the claims 1 to 14, characterized in that the cup element (40) has at least two recesses (49) arranged diametrically on the front side opposite to the hip bone (20), for the accommodation of a rotating tool (72).

16. The acetabular cup according to one of the claims 1 to 15, characterized in that the inner cup (50) comprises a cutout on the side facing the hip bone or a through-hole (55) which is opened to the outer cup and has an undercut (57) and on the outside of the inlay (60), a lock portion (67, 69) is provided coacting with the cutout and the undercut (57), respectively.

17. The acetabular cup according to claim 16, characterized in that the inlay (60) made of plastic material comprises an approximately cylindrical trunnion (67) which has a plurality of noses (69) spaced in circumferential direction and arranged on the outside of the trunnion, the noses coacting with the undercut (57) in the through-hole (55).

18. The acetabular cup according to one of the claims 1 to 17, characterized in that the acetabular cup (21) is combined with a femur portion (3) of a joint endoprosthesis or a hip joint endoprosthesis, with an internal space (12) being provided within a shaft (4) of the femur portion (3), and openings (14, 15) being provided between the outside of the shaft and the internal space thereof, the openings (14, 15) being solely arranged in the frontal side (10) and/or the dorsal side (11) of the shaft (4), the openings (14, 15) and the internal space (12) ensuring the introduction and the holding of spongiosa (16) or loose bone material into and in the hollow internal space (12) during the operation by means of the shape and sizes of the openings and the internal space and, thereafter, ensuring growth thereinto of new strong bone material, with the shaft (4) being a cast component which has a solid, comparatively thick wall (17) ensuring also a support for the introduced bone material within the openings (14, 15), and with the profile of the outside of shaft (4) being adapted to be fitted to the associating internal profile of the marrow canal (2).

## Revendications

1. Pièce formant coque d'articulation (21) pour une prothèse d'articulation, en particulier pièce formant coque acétabulaire d'une endoprothèse d'articulation de hanche, comportant un élément en coquille (32, 40 et 50) fixé dans l'os et qui présente, sur son côté articulation, un renfoncement en forme de cuvette, par exemple en forme de demi-sphère, pour recevoir une coupelle de glissement (25), ou un inlay (60) pour l'articulation, caractérisée en ce que l'élément en coquille (32; 40 et 50) est construit à partir d'au moins deux parties de coquille (22, 24; 40, 50) disposées à une certaine distance radialement l'une de l'autre avec un espace intermédiaire libre ( 27; 54 ), parties de coquille dont des ouvertures (26; 43, 44) sont prévues au moins dans la partie de coquille tournée vers l'os de la hanche (20) (coquille intérieure 22, 40), ouvertures dont le nombre, la répartition, la forme et la grandeur permettent, pendant l'opération, d'introduire, dans l'espace intermédiaire (27; 54), de la matière spongieuse ou de la substance osseuse détachée (16) et, ensuite, la croissance, à l'intérieur, de nouvel le substance osseuse compacte.

2. Pièce formant coque d'articulation suivant la revendication 1, caractérisée en ce que l'élément en coquille (32; 40 et 50) est formé d'une seule pièce.

3. Pièce formant coque d'articulation suivant la revendication 1, caractérisée en ce que les parties de coquille (22, 24, 40, 50) sont formées d'une seule pièce, et enfoncées, ajustées, les unes dans les autres, et sont ensuite reliées les unes avec les autres, de préférence par soudure.

4. Pièce formant coque d'articulation suivant l'une quelconque des revendications 1 à 3, caractérisée en ce qu'entre les deux parties de coquille (22, 24), sont, de préférence, prévues des tiges radiales d'appui et de maintien à distance.

5. Pièce formant coque d'articulation suivant l'une quelconque des revendications 1 à 4, caractérisée en ce que les ouvertures (26; 43) dans la partie de coquille (22; 40) tournée vers l'os sont circulaires.

6. Pièce formant coque d'articulation suivant l'une quelconque des revendications 1 à 4, caractérisée en ce que les ouvertures (26; 44) dans la partie de coquille (22; 40) tournée vers l'os sont allongées longitudinalement dans la direction périphérique.

7. Pièce formant coque d'articulation suivant la revendication 6, caractérisée en ce que plusieurs séries, dirigées suivant la direction périphérique, d'ouvertures (44), allongées longitudinalement, sont prévues et en ce que les ouvertures (44) d'une série sont disposées décalées par rapport à celles de l'autre série.

8. Pièce formant coque d'articulation suivant la revendication 6, caractérisée en ce que plusieurs séries, dirigées suivant la direction périphérique, d'ouvertures (44′), allongées longitudinalement, sont prévues et en ce que les ouvertures (44′) de toutes les séries sont dirigées suivant un axe.

9. Pièce formant coque d'articulation suivant l'une quelconque des revendications 1 à 8, caractérisée en ce qu'entre les ouvertures (43, 44, 44′), des sections (45, 45′) d'un filetage auto-taraudant sont formées sur la face extérieure de l'élément de coquille.

10. Pièce formant coque d'articulation suivant la revendication 8 et la revendication 9, caractérisée en ce que le filetage est interrompu par des évidements de telle façon que les sections de filetage se terminent, dans les évidements, suivant une ligne (46).

11. Pièce formant coque d'articulation suivant l'une quelconque des revendications 1 à 10, caractérisée en ce que les parties de coquille (22, 24) ont la forme d'une partie de sphère.

12. Pièce formant coque d'articulation suivant l'une quelconque des revendications 1 à 10, caractérisée en ce que les parties de coquille (22, 24) ont la forme d'un élément tronconique.

13. Pièce formant coque d'articulation suivant l'une quelconque des revendications 1 à 12, caractérisée en ce que les parties de coquille (22, 24; 40, 50) sont disposées concentriquement.

14. Pièce formant coque d'articulation suivant l'une quelconque des revendications 1 à 13, caractérisée en ce que la partie de coquille (22) tournée vers l'os est réalisée sous la forme d'un treillis grillagé réticulaire (33), lequel remplit, au moins partiellement, l'espace intermédiaire (27) et peut être traversé par la croissance de l'os et au travers duquel l'os peut se développer , et dont les différents éléments de grillage ou du treillis réticulaire prennent solidement appui les uns contre les autres, et de préférence sont reliés ensemble solidement aux points de croisement.

15. Pièce formant coque d'articulation suivant l'une quelconque des revendications 1 à 14, caractérisée en ce que l'élément de coquille (40) présente, sur la face frontale située du côté opposé à l'os de la hanche (20), au moins des évidements (49) diamétralement opposés pour recevoir un outil de mise en rotation (72).

16. Pièce formant coque d'articulation suivant l'une quelconque des revendications 1 à 15, caractérisée en ce que la coquille intérieure (50) présente, du côté tourné vers l'os de la hanche, un évidement ou un passage (55) ouvert vers la coquille extérieure (40), avec une contre-dépouille (57) et l'inlay (60) présente sur la face externe une section de retenue (67, 69) qui coopère avec l'évidement, en particulier la contre-dépouille (57).

17. Pièce formant coque d'articulation suivant la revendication 16, caractérisée en ce que l'inlay (60) constitué de matière plastique présente un pivot (67) sensiblement cylindrique, qui présente, sur la face extérieure, plusieurs ergots (69) répartis suivant la direction périphérique, qui coopèrent avec la contre-dépouille (57) prévue dans le passage (55).

18. Pièce formant coque d'articulation suivant l'une quelconque des revendications précédentes 1 à 17, caractérisée en ce que la pièce formant coque d'articulation (21) est combinée avec une pièce de fémur (3) d'une endoprothèse d'articulation, en particulier d'articulation de hanche, un espace intérieur (12) étant prévu dans la tige (4) de la pièce de fémur (3), ainsi que des ouvertures (14, 15) entre la face extérieure de la tige et son espace intérieur, étant entendu que les ouvertures (14, 15) sont disposées seules dans la face frontale (10) et/ou dans la face dorsale de la tige (4), les ouvertures (14, 15) et l'espace intérieur (12) assurant, pendant l'opération, l'introduction et le maintien de matière spongieuse (16) ou de substance osseuse détachée dans l'espace intérieur creux (12) et, ensuite, la croissance, à l'intérieur, de nouvelle substance osseuse compacte, étant entendu que la tige (4) est constituée d'une pièce moulée de fonderie, dont la paroi (17) épaisse, comparativement massive, assure à la substance osseuse introduite un maintien et un appui même à l'intérieur des ouvertures (14, 15), et que la face extérieure de la tige (4) est réalisée, avec un profil ajusté étroitement au profil intérieur correspondant du canal médullaire (2).

Fig. 4

Fig. 1

Fig. 2

# Fig. 5

# Fig. 3

# Fig. 6

Fig. 7

43 44 40

41

VIII VIII

43

43 44

Fig. 8

57 55 52 41 40

54 45

44 44

45 56 58 59

43 51 43

VIII a

50 42 53

Fig. 8a

40

49

Fig. 9

40'

45'

44' 46 47

Fig. 9a

45" 75

Fig. 10

Fig. 11

Fig. 12

Fig. 13

13